(19) Europäisches Patentamt
European Patent Office
Office européen des brevets

(11) EP 2 025 242 A1

(12) EUROPEAN PATENT APPLICATION

(43) Date of publication:
18.02.2009 Bulletin 2009/08

(51) Int Cl.:
*A23K 1/00* (2006.01)

(21) Application number: 07014605.5

(22) Date of filing: 25.07.2007

(84) Designated Contracting States:
AT BE BG CH CY CZ DE DK EE ES FI FR GB GR HU IE IS IT LI LT LU LV MC MT NL PL PT RO SE SI SK TR
Designated Extension States:
AL BA HR MK RS

(71) Applicant: Universität Hohenheim
70593 Stuttgart (DE)

(72) Inventors:
• Makkar, Harinder P.S.
70599 Stuttgart (DE)

• Becker, Klaus
72622 Nürtingen-Reudern (DE)
• Goel, Gunjan
Haryana (IN)

(74) Representative: Elbel, Michaela
Rothkopf Theobald Elbel
Patentanwälte, Partnerschaft
Isartorplatz 5
80331 München (DE)

(54) **Feed and feed additive for herbivores, and method for manufacturing the same**

(57) The present invention concerns a feed or feed additive for herbivores, which comprises of a *Moringaceae.* Further, the invention comprises a kit for the treatment and/or prevention of gastro-intestinal diseases and/or disorders of herbivores. Further, methods for manufacturing a feed or feed additive and a method for increasing livestock productivity of herbivores is encompassed.

A

B

Figure 1

EP 2 025 242 A1

**Description**

**Field of the invention**

The present invention relates generally to feed and feed additives, especially comprising components originally isolated from plants, that can be used to increase livestock productivity, and to methods for manufacturing a feed or feed additive for herbivores comprising at least one component of *Moringa*.

**Background of the invention**

Methane is a relatively potent greenhouse gas with a global warming potential. When averaged over 100 years each kilogram of methane warms the earth 25 times as much as the same mass of $CO_2$. While emission from power plants, auto tailpipes and forest fires have long been blamed for warming the planet, the innards of livestock, including sheep, goats, cattle etc., are now being recognized as significant contributors as well. The world animal herds number about 1.3 billion cattle and 1.1 billion sheep. In the United States cattle become so numerous that there are two animals for every five people (Polakovic G., 2003). Methane concentration in the atmosphere has more than doubled to 800 million tons annually in the past century, with agriculture methane as the greatest source accounting for about 35-40 % of the total anthropogenic methane emissions. Of the agriculture methane emissions about two-thirds come from enteric fermentation and one-third from livestock manure (Moss A. et al., 2000).

Fermentation processes carried out by rumen microbes can be considered as an anaerobic fermentation of carbohydrates and proteins to acetate, $CO_2$ and ammonia giving concomitant reduced co-factors, like NADH. These reduced co-factors are reoxidized to NAD by non-oxygen electron acceptors as $CO_2$, C2 molecule or pyruvate. In the rumen, production of methane is the major way of hydrogen elimination by the following reaction:

$$CO_2 + 4\,H_2 \rightarrow CH_4 + 2\,H_2O$$

If $CO_2$ is replaced by pyruvate, propionate is formed with some energy gain (Moss, A. et al., 2000).

The methane producing reaction is carried out by archaea known as methanogens that are normal components of the rumen microbial ecosystem. The methanogens that were identified from the rumen belong to the genera *Methanobrevibacter, Methanobacterium, Methanomicrobium* and *Methanosarcina* (Wolin, M. et al., 1997). The collaboration between fermentation and hydrogen utilising bacteria is called "interspecies hydrogen transfer" facilitated by some physical associations of microbes (Moss A. et al., 2000).

Because ruminants are the major anthropogenic source of methane, reducing its emission is a vital component of world wide campaigns, like the Kyoto protocol to prevent global warming. Various strategies were suggested for reducing ruminal methanogenesis.

**1. Modification of feeding practices**

The type of feed consumed has significant effects on the proportion of energy emitted as methane. For example, more concentrate feeding lowers the pH value in the rumen and methane bacteria are inhibited at lower pH values. In addition, decreased methanogenesis is accompanied by higher proportions of propionate due to interspecies hydrogen transfer. Accordingly, dietary interventions causing shifts in volatile fatty acids in favor of propionate also result in lowering of methane production. Such a decreased acetate:propionate ratio can be achieved by increasing the proportion of easily fermentable carbohydrates as well as by physical treatment of the ration, like grinding or treatment with heat. Increasing the level of feeding with hay-concentrate based diets also result in reduced methane production. In addition, low frequencies of feeding tend to increase propionate and decrease methane production associated with lowering rumen protozoa and fungi (van Nevel C. and Demeyer D., 1996; Sharma R., 2005).

**2. Feed additives**

Another approach for suppression of methanogenesis is to add live microbial supplement called probiotics to the feed that beneficially affects the host by improving its intestinal microbial balance. Some aerobic yeasts or fungi as *Saccaromyces* or *Aspergillus spp.* in small amounts were reported to show positive effects (Malik R. and Sirohi S., 2000).

Several chemical compounds such as chlorinated methane analogues have been reported to be toxic to methanogens. However, ruminal microbial populations *in vivo* adapt or degrade many of these compounds and positive effects on methane reduction were rarely observed (Sharma R., 2005).

For many years antibiotics have been used in animal production as growth promoting substances. Indeed, antibiotics inhibit methane production by direct or indirectly interfering with the methanogens. Ionophores are to be used

**EP 2 025 242 A1**

with caution as these antibiotics were found to depress fibre digestion and inhibit protozoan growth. In addition, ruminal bacteria appear to develop resistances and absorbed antibiotics accumulate in the animal body affecting animals as well as humans consuming their milk and meat (Newbold C. et al., 1993).

Organic acids, including malic and fumaric acids, were shown to decrease methane formation by acting as an alternative hydrogen acceptor. Although they were found to be effective hydrogen sinks forming propionate instead of methane, their widespread use in the field is problematic, because their acidic properties restrict the quantity which can be fed (Wallace R. et al., 2006).

While control of rumen methanogenesis may be achieved by a range of inhibitors, efficacy of many agents declines with continued or repeated use. Furthermore they are toxic and cause disruption of feed digestion. In addition, there is growing concern about using chemicals in animals destined for human consumption.

Thus, there is a need for an alternative bioactive substance that inhibits methane production in ruminant animals.

The solution to said technical problem is achieved by providing the embodiments characterized in the claims, and described further below.

## Summary of the invention

The present invention is directed to a feed and feed additive for herbivores, wherein the feed and feed additive comprises at least one component of a *Moringaceae*.

Furthermore the invention concerns a kit comprising at least one component of a *Moringaceae* for the treatment and/or prevention of gastro-intestinal diseases and/or disorders of herbivores.

The present invention is also directed to a method for manufacturing a feed for herbivores comprising the steps of:

- providing at least one basic feed ingredient;
- obtaining at least one component of a *Moringaceae*; and
- mixing at least one component of a *Moringaceae* with at least one basic feed ingredient;
  and to a method for manufacturing a feed additive for herbivores comprising the steps of:
- providing at least one feed additive constituent;
- obtaining at least one component of a *Moringaceae*; and
- mixing at least one component of a *Moringaceae* with at least one feed additive constituent.

In addition, the present invention is related to a method for increasing livestock productivity and/or to decrease methane emission from livestock of herbivores comprising the steps of:

- providing a feed or a feed additive comprising at least one component of a *Moringaceae*; and
- feeding the feed or the feed additive to herbivores.

Furthermore the invention concerns the use of a feed or feed additive comprising at least one component of a *Moringaceae* to increase livestock productivity and/or to decrease methane emission from livestock.

## Brief description of the figures

Figure 1 illustrates the effect of *Moringa oleifera* on methane production on a volume basis. The methane production in Figure 1A is compared with the methane reduction in Figure 1B.

Figure 2 illustrates the effect of *Moringa oleifera* on methane production on a truly degraded matter basis. The methane production in Figure 2A is compared with the methane reduction in Figure 2B.

Figure 3 illustrates the effect of *Moringa oleifera* on microbial shift.

## Detailed description of the invention

The present invention is directed to a feed and feed additive for herbivores, wherein the feed comprises at least one component of a *Moringaceae*.

The present invention provides a new feed and feed additive with a surprising effect of inhibiting methanogenesis. The substance that is responsible for the inhibition is a component of a *Moringaceae*.

The term "*Moringaceae*" as used herein refers to a plant family of the order Brassicales. All 13 species of this family are trees that grow in tropical and sub-tropical climates. They are very popular plants used for food and traditional medicine. Some species, like *Moringa drouhardii* and *Moringa hildebrandtii*, are bottle trees characterized by their massive, water storing trunks.

The term "feed" as used herein refers to fodder or animal feed, i.e. foodstuff that is used to feed animals.

The term "feed additive" as used herein refers to feed supplements that provide additional nutrients and/or micro nutrients to the feed.

The feed and feed additives can be embodied in the same way as the manufactured feed and feed additives according to the invention.

The term "herbivores" as used herein refers to animals that eat exclusively or primarily plant matter represented for example by the family *equidae*. Herbivores are not restricted to defined animal classes, but are represented in a plurality of orders in different classes, especially mammals.

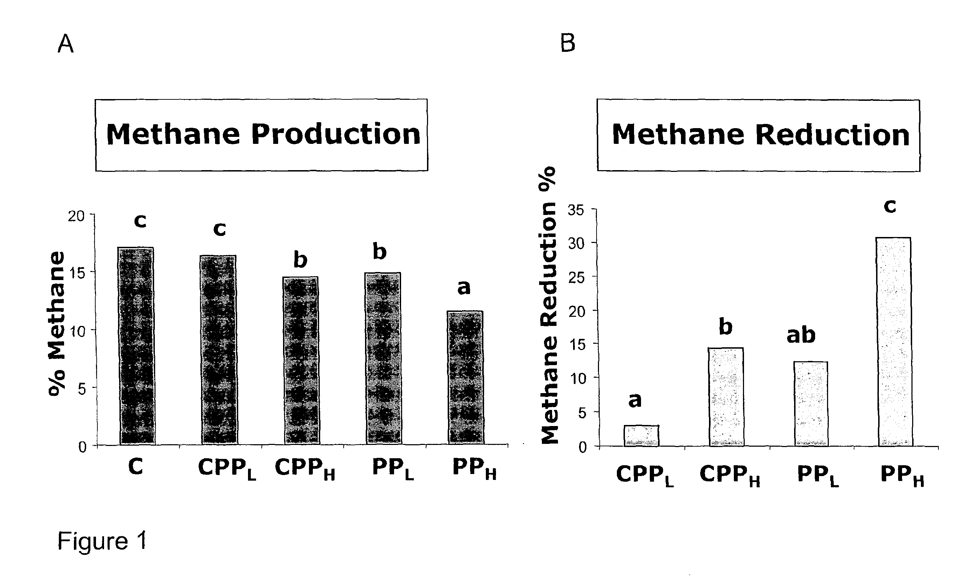

The present invention is based on the findings that crude proteins and purified proteins extracted from a *Moringaceae* show strong anti-methanogenic activity. The crude protein preparation from defatted *Moringa oleifera* seeds decreased methane production by about 3.0% to 14% on a volume basis (MR%) and by about 4% to 20% per unit degraded matter ($MR_{TD}$%) in incubation medium compared to controls (Figure 1, Table 1) using the *in vitro* gas production method of Menke and Steingass (1988). The effect of the purified, pure protein is more prominent with a decrease of methane production by about 12.4% and 30.8% on a volume basis and by about 16.4% and 43.8% per unit degraded matter (Figure 1, Table 1).

Due to these effects the feed or feed additive according to the invention has the advantage to improve the efficiency of digested energy utilisation by herbivores, because methane production represents a loss of feed energy that varies from 5% to 15% of gross energy intake depending of the type of diet. This is supported by data herein (Table 1) showing that the crude protein increased the partition factor from 3.2 (control) up to 3.5. The partition factor is an index for efficiency of microbial protein synthesis, i.e. the higher the partition factor, the higher is the efficiency. The purified, pure protein increased the partition factor from 3.4 to 4.

A further advantage of the anti-methanogenic effect of the feed or feed additive according to the invention is the reduction of greenhouse gas emission from livestock. This contributes to the efforts to reduce global warming, which is getting more and more important.

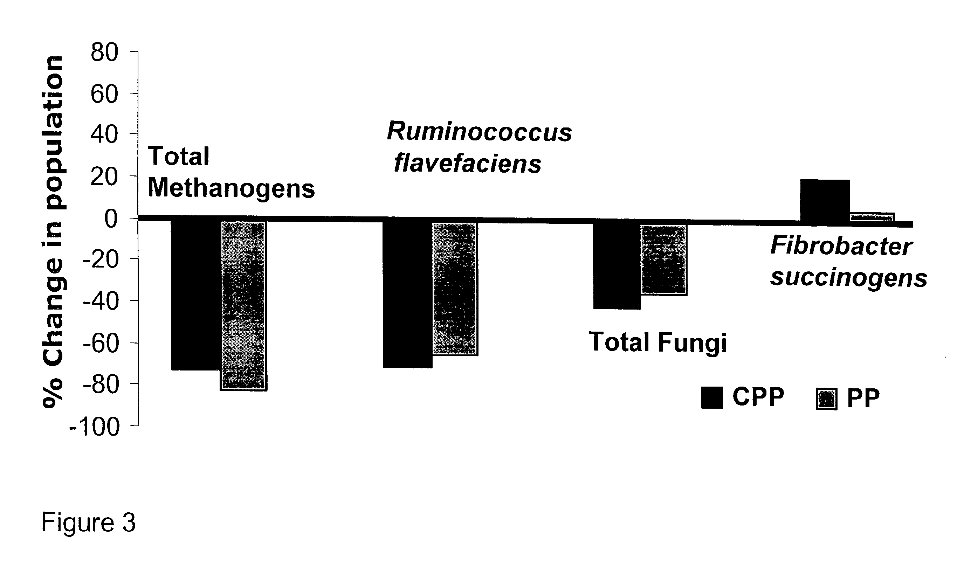

The anti-methanogenic activity is possibly based on the ability of the protein extract to influence the microbial population and to reduce the methanogens (Fig. 3), but the mechanism of action is still not clear.

Methanogens are considered to be a very old group of organisms that are found in extreme ecosystems. They have been identified only by the kingdom *Archaea*, a group being distinct from both eukaryotes and bacteria. Methanogens perform a special form of anaerobic respiration, called methanogenesis. They can not use and exploit oxygen due to the lack of catalase and superoxide dismutase, but they are rapidly killed in the presence of oxygen. Instead, methanogens use carbon molecules, like carbon dioxide or acetic acid as terminal electron acceptors and hydrogen as electron donator, called carbonate respiration.

Methanogens are not only distinguished from bacteria by their metabolic activities, but also by their molecular morphology. Their cell wall does not show a peptidoglycan structure, but a coating of proteins, polypeptides or polysaccharides, like uronic acids, amino acid saccarides and neutral saccarides. Due to this, they are insensitive to antibiotics like penicillin, cephalosporin or cycloserine. The cytoplasmic membrane contains lipids from glycerol ethers and isopranoide hydrocarbons instead of glycerol esters. Compared to eubacteria, rRNA sequences and RNA polymerases of methanogens are very different and protein synthesis at ribosomes is insensitive to antibiotics like rifampicin and chloramphenicol.

In a preferred embodiment the components of a *Moringaceae* for the feed or feed additive comprise all constituents of the plant including barks, roots, seeds, wood, branches, leaves, fruits and flowers as well as the complete plant itself, e.g. embryos, seedlings etc.. The components are used as entire or chopped components. The term "chopped components" as used herein means components in any size from coarse lumps to fine powder. Therefore, they may be prepared for mixing with other feed ingredients. The components of a *Moringaceae* also comprise natural extracts from any of its components including sap, gum, exudates, oil etc..

*Moringaceae,* particularly *Moringa oleifera*, are very popular plants. Immature green pods, leaves and seeds are highly nutritious, and are therefore considered as significant sources of beta-carotene, vitamin C, iron, potassium, protein, oil and other effective secondary plant substances. Many ingredients of *Moringaceae* are unknown, but could be responsible for the frequent use of the plant in traditional medicine. These advantages make the components of a *Moringaceae* ideal to be contained in the feed or feed additive of the present invention.

In a more preferred embodiment the components of a *Moringaceae* for the feed or feed additive are natural proteins extracted from any of its components especially crude and purified natural proteins. As herein exemplified the proteins were prepared by acetone precipitation from defatted *Moringa oleifera* seeds and subsequently quantified using SDS-PAGE. It will be understood that there is no limitation to the method for obtaining and purifying extracts and the person skilled in the art may choose the appropriate method within the broad field of biochemistry.

The naturally extracted proteins of a *Moringaceae* have the advantage that the anti-methanogenic activity is not affected by heat treatment, e.g. 100 °C for 30 min. This heat stability is a useful property for manufacturing the feed or feed additive, because heating is often necessary for e.g. sterilization or pasteurisation during the manufacturing procedure.

In the common meaning proteins are large organic compounds, also called polypeptides, made of different amino acids that are arranged in a linear chain and joined together by peptide bonds between the carboxyl and amino groups of adjacent amino acid residues. Most proteins fold into unique 3-dimensional structures, namely primary, secondary, tertiary and quaternary structures. Peptides are short molecules also formed from the linking of various α-amino acids. Usually peptides are shorter than proteins, however, there is no exact definition where a peptide or an oligopeptide ends and a polypeptide or protein begins. There are several different conventions to determine these, but all these conventions have drawbacks and none is well defined. Due to this, the term "protein" as used herein refers to proteins, polypeptides and peptides of arbitrary length with or without 3-dimensional structures.

The components of a *Moringaceae* according to the invention also comprise artificially synthesised proteins that are identical to or derived from natural proteins. They are chemically synthesised by connecting amino acids or peptide fragments step-by-step for elongation or by chemical ligation (Nilsson B. et al., 2005).

The term "recombination" as used herein refers to the recombination of genetic information in any *in vivo* or *in vitro* system. Expression by a recombinant system is another possibility for artificial protein synthesis. After extraction, DNA or RNA of a *Moringaceae* can be optionally modified by different molecular tools, for example by exchanging promoters and insertion of enhancer sequences for increased expression or by transcription of RNA into DNA. Nucleic acids can also be chemically synthesised with sequences that are identical or similar to naturally occurring sequences of a *Moringaceae.* The obtained nucleic acids are transfected into suitable cloning vectors as plasmids, viral and eukaryotic vectors. Subsequently, the recombined DNA is integrated into the DNA of desired host organisms which then express the encoded product. These hosts may comprise prokaryotic and eukaryotic organisms. The recombinant system can be established on the basis of an *ex vivo* system as a PCR reaction, i.e. that enzymes, substrates and other necessary molecules are added to the recombined genetic information outside an organism to gain the encoded recombinant protein or recombinant factor. In addition, the recombinant system can be established on the basis of an *in vitro* system with vectors or simple organisms. For example, bacteria as *E. coli*, viruses as phages or yeasts are advantageous, because expressed proteins are easily accessible. Further the recombinant system can be established on the basis of an *in vivo* system with complex organisms. For example, plants are advantageous, because they can serve themselves as feed together with the desired protein making protein extraction unnecessary.

By means of the above recombined systems not only proteins, but also factors according to the present invention are generated by recombination. As used herein the term "factors" refers to compounds that are metabolically related in a direct or indirect way to proteins that are expressed by recombination.

The term "*Moringa*" as used herein refers to the only genus of the plant family *Moringaceae. Moringa*, which is preferably used herein, comprises 13 species, all of which are trees that grow in tropical and sub-tropical climates. Any of these species may be used in the present invention.

In another preferred embodiment of the present invention a certain variety of the genus *Moringa* is used, namely *Moringa oleifera. Moringa oleifera* is a tree that originates from India and is cultivated throughout the tropics. Other English names for *Moringa oleifera* are "drumstick tree", "horseradish tree" and "ben oil tree". This tree has the advantages that it tolerates poor soil, grows fast and is drought-resistant making it ideal for being cultivated. The roots of *Moringa oleifera* contain the alkaloid spirochin, a fatal nerve paralyzing agent, and should therefore be used very carefully as a condiment. In Siddha medicine, the drumstick seeds are used as a sexual virility drug for treating erectile dysfunction in men and prolonging sexual activity in women. The seeds yield up to 40% edible oil containing a high concentration of behenic acid that can be used in cooking, cosmetics and lubrication. The leaves are highly nutritious with high contents of vitamin and minerals. In addition, *Moringa oleifera* is of interest because of its production of different glucosinolates, like benzyl glucosinolates and its anti-rheumatoid effects. Other preferred genera are *Moringa concanensis, Moringa peregrine, Moringa ovalifolia, Moringa drouhardii* or *Moringa hildebrandtii.*

In a preferred embodiment of the present invention the herbivores are ruminants. The term "ruminants" means any hooved animal of the suborder *Ruminantia* and the order *Artiodactyla* that digest its food in two steps, first by eating the raw material and regurgitating a semi-digest form known as cud, then by chewing and eating the cud, a process called ruminating. Ruminants comprise cattle, goats, sheep, llamas, giraffes, bisons, buffalo, deer, wildebeest and antelope. Llamas and camels are members of *Tylopoda*, another suborder of *Artiodactyla*.

In a particularly preferred embodiment of the present invention, the feed or feed additive comprises proteins that are encoded by sequences selected from the group consisting of SEQ ID NO: 1 and 2 and derivates thereof. The term "SEQ ID NO: 1 and/or 2" as used herein also refers to derivates of the sequences. The derivates may have an identity of 80 %, preferably 90 %, most preferably > 95 % of SEQ ID NO: 1 and/or 2. The protein of SEQ ID NO: 1 is a natural protein extracted from *Moringa oleifera.* The protein of SEQ ID NO: 2 is the corresponding recombinant protein that differs from the natural protein by two amino acid residues. Cysteines at positions 13 and 23 of SEQ ID NO: 1 are replaced in SEQ ID NO: 2 by glycine and glutamine, respectively. The recombinant protein of SEQ ID NO: 2 was expressed in *E. coli* and subsequently purified, sequenced and identified by its flocculating activity. The proteins of SEQ ID NO: 1 and 2 are polycationic and migrate at 6.5 kDa on SDS-PAGE with an isoelectric point above 10 and contain 60 amino acids with a high content of glutamine, arginine and proline.

The factors generated by recombination comprise DNA, RNA, and PNA in the up stream direction. In the down stream direction the factors comprise products of enzymatic reactions, like phosphorylated or dephosphorylated compounds etc.; co-factors, like non-protein co-enzymes, e.g. biotin; receptors and ligands, like hormones, transmitters, electron acceptors and donators etc.; glycoproteins; lipoproteins and small molecules, including organic molecules, like amino acids or inorganic molecules, like ions.

In a further aspect the present invention relates to a kit comprising at least one component of a *Moringaceae* for the treatment and/or prevention of gastro-intestinal diseases and/or disorders of herbivores.

The terms "gastro-intestinal diseases and/or disorders" as used herein refers to diseases and/or disorders concerning the digestive organs including secondary effects from digestive organs to non-digestive organs and *vice versa*.

There are several reasons for changes in the microbial ecosystems of herbivoral intestinal tract. Different feed is digested by different classes of bacteria, and provision must be made to allow a new population of bacteria to establish when changes are made in the ration. Diseases or disorders especially of the gastrointestinal tract and treatment with drugs are further reasons for changes in the microbial ecosystem. As shown in Figure 3, components of a *Moringaceae* possess the property to shift the microbial ratio. A crude protein preparation causes reduction of 73%, 71 % and 42% of methanogens, *Ruminococcus flavefaciens* and ruminal fungal population, respectively and an increase of 21% in the population of *Fibrobacter succcinogenes* compared to controls. Due to this shifting property, the components of a *Moringaceae* have the potential to rebalance the ratio of microbes in the herbivoral intestinal tract.

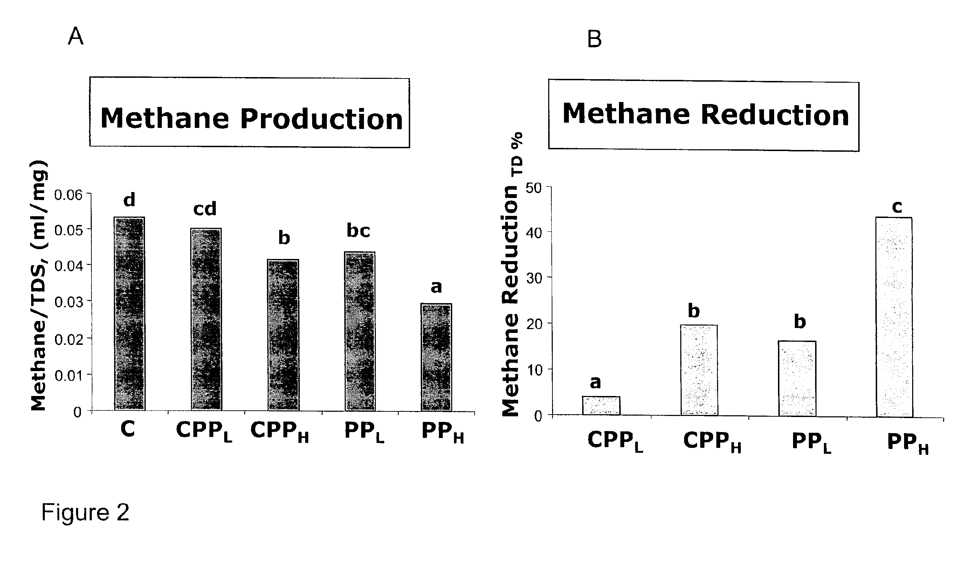

The above described anti-methanogenic activity of components of a *Moringaceae* (see also Fig. 1, 2, Table 1) is another property that gives a kit comprising a component of a *Moringaceae* the ability to treat and/or prevent gastro-intestinal diseases and/or disorders of herbivores. Colic, bloats and flatulence, to which ruminants are subject, are examples of gastro-intestinal diseases and/or disorders that are accompanied by excessive gas formations in frothy or free gas types. Due to the reduction of methane based gas and foam formation by components of a *Moringaceae*, a kit including components of a *Moringaceae* can be used for prevention/treatment of gastro-intestinal diseases and/or disorders of herbivores.

A further aspect of the invention is a method for manufacturing a feed for herbivores comprising the steps of:

- providing at least one basic feed ingredient;
- obtaining at least one component of a *Moringaceae*; and
- mixing at least one component of a *Moringaceae* with at least one basic feed ingredient.

The term "basic feed ingredients" as used herein refers to products that are manufactured from various raw materials; for examples see below.

The manufactured feed can be a complete feed that provides all the nutrients required daily, concentrates that provide a part of the ration, e.g. in the form of protein or carbohydrate concentrates, or premixes that comprise essential feed ingredients for blending into commercial or home-made feed.

The physical type of the manufactured feed is not restricted and ranges from oil and press cake, pellets and crumbles to coarsely granulate and fine grinded powder. It may be manufactured in a liquid or dry form that can be optionally mixed with water or other liquids.

A further aspect of the invention is a method for manufacturing a feed additive for herbivores comprising the steps of:

- providing at least one feed additive constituent;
- obtaining at least one component of a *Moringaceae*; and
- mixing at least one component of a *Moringaceae* with at least one feed additive constituent.

The term "feed additive constituent" as used herein refers to constituents of various feed supplements that provide additional nutrients and/or micro nutrients; for examples see below.

In addition, in a preferred embodiment of the invention the feed additive is manufactured in a form that can be directly administered to the animal, e.g. by oral application. Alternatively, it is manufactured for admixing to feed. Other routes of application, like injections or intranasal application, are also encompassed, but oral application is preferred.

Another aspect of the invention is a method for increasing livestock productivity of herbivores comprising the steps of:

- providing a feed or a feed additive comprising at least one component of a *Moringaceae*; and

- feeding the feed or the feed additive to herbivores.

The basic feed ingredient as defined above is manufactured from raw material selected from the group consisting of cereals, especially oats, sorghum and wheat, buckwheat, millet, corn, potatoes, vegetables, legumes, especially soy beans, brassicas, manioc, alfalfa, oily seeds, fish, fibres and hulls as grass and hay etc. including their intermediates and different additives according to the specific requirements of the target animal.

The feed additive constituent as defined above is selected from the group consisting of minerals i.e. inorganic compounds, like calcium and potassium; organic and inorganic trace elements, e.g. trace metals, like copper, iron and zinc; vitamins, like vitamin E, D, B, C; enzymes; co-enzymes; micro organisms, called probiotics, like yeasts; essential amino acids and fatty acids; immuno modulatory substances that stimulate or inhibit the immune system in a specific or non-specific way, like antigens, leukines and glucocorticoides and mycotoxin binding and deactivation agents, like bentonite clay and enzymes. The feed additives according to the present invention can be manufactured and provided in the same administration forms as described above for the feed.

In a preferred embodiment of the above described methods of the invention, the component of the *Moringaceae* is selected from the group consisting of chopped components, seeds, natural proteins extracted from a *Moringaceae,* chemically synthesised proteins, proteins expressed by recombination and factors generated by recombination.

In another embodiment of the above described methods of the invention, *Moringaceae* is *Moringa*, preferably *oleifera*.

In another embodiment of the invention the manufactured feed or feed additive according to the invention is provided in dosage units for e.g. daily ratios per animal or per kilogram of animal, or alternatively provided in a bulk form for variable dosage.

A further aspect of the invention relates to the use of a feed or feed additive comprising at least one component of a *Moringaceae* to increase livestock productivity and /or decrease methane emission form livestock.

This use is based on the effect that reduced methanogenesis improves the efficiency to utilize digested energy by animals due to the reduced loss of energy in the form of methane. Therefore, the feed or feed additive according to the invention is ideal for feeding herbivores, especially ruminants.

In a preferred embodiment of the invention the feed additive is directly administered to the animal, e.g. by oral or intranasal application to increase livestock productivity. Alternatively, the feed additive is administered by admixing to feed.

All individual elements of the above described embodiments can be combined independently. Modifications of the embodiments as estimated by the person skilled in the art are within the scope of the invention.

The following specific examples are provided for purposes of illustration only and are not intended to limit the scope of the invention.

**EXAMPLES**

**1. Methods**

**1.1 Protein fractions used for the study**

An acetone precipitated fraction of defatted *Moringa oleifera* seed material was prepared. It was resolved using SDS-PAGE, and the protein bands were quantified using an image analyzer. A small protein of 6.5 kDa revealed a major protein band of 50-60% of the total protein. The crude protein preparation obtained by acetone fractionation and the purified protein of 6.5 kDa were used in the study.

**1.2 *In vitro* rumen incubation system and methane measurement**

2.5 and 5 mg of crude protein preparation per ml incubation medium along with 380 mg hay substrate in 40 ml incubation medium containing rumen microbes in bicarbonate based buffered system, pH 6.8 were incubated in 100 ml capacity calibrated syringes using the *in vitro* gas production method of Menke and Steingass (1988). The effect of 1.25 and 2.5 mg purified protein of 6.5 kDa per ml of the incubation medium was also evaluated.

The corresponding blank contained buffered medium without hay substrate, but the same amount of protein. The control syringe consisted of buffered medium containing substrate (hay) without any addition of protein. The syringes were incubated at 39°C for 24 h. After fermentation for 24 h, various determinations were made: total gas, $CH_4$, true degradability of the substrate, short chain fatty acids and partitioning of the substrate-carbon to gases and microbial mass.

### 1.3 Gas and methane determination

After 24 h of incubation, the total gas was recorded from the calibrated scale on the syringe. Methane was measured using an infra-red (0 to 30% range) methane analyser (Pronova Analysentechnik GmbH & Co KG, Berlin, Germany) calibrated against 10.6 % methane.

### 1.4 Determination of true dry matter degradability

The contents of the syringes after 24 h of incubation were dissolved with neutral detergent solution and undissolved feed was recovered on crucibles, washed and dried at approximately 90°C for 16 h. The truly degraded substrate in 24 h was calculated by subtracting this value from the dry matter incubated in the syringe (Blümmel M., et al., 1997).

### 1.5 Short chain fatty acid

The short chain fatty acids in the medium obtained from the fermentation system were determined by using a gas chromatography, according to the procedure of Maiworm K. (1994).

### 1.6 Calculations

The measurements of total gas, methane and truly degraded substrate were used for calculation of methane reduction and the partitioning of nutrients. Net gas (NG in ml) was calculated from the differences of gas in the test syringe and the corresponding blanks, and similarly net methane produced (NM in ml) was determined by subtracting the methane in the blank syringe from that in the test syringe. The percentage of methane is calculated by the formula:

$$Met\% = \frac{NM}{NG} \times 100$$

The effect of the treatment, as percent decrease in methane (MR%), was calculated as:

$$\frac{(\text{Percent methane in the control} - \text{Percent methane in the test sample}) \times 100}{\text{Percent methane in the control}}$$

The effect of the treatment, as percent decrease in methane based on truly degraded substrate ($MR_{TD}$), was calculated in the same way from the values for net methane produced per mg truly degraded dry matter and the values for the control, wherein the values of the control were taken as 100%.

The partition factor (PF), which is a measure of efficiency of microbial protein synthesis (Blümmel M. et al., 1997), was calculated as:

$$\frac{\text{truly degraded substrate}}{\text{ml of produced NG}}$$

### 2. Results and detailed description of the figures

A crude protein preparation from defatted *Moringa oleifera* seeds in concentrations of 2.5 mg and 5 mg per ml of incubation medium decreased methane production by about 3.0% and 14.4% on a volume basis (Figure 1, Table 1) and by about 4% and 20%, respectively, when expressed as "ml methane produced per unit mg truly degraded substrate" (Figure 2, Table 1). Pure protein of *Moringa oleifera* seeds in concentrations of 1.25 mg and 2.5 mg per ml of incubation medium decreased methane production by about 12.4% and 30.8% on a volume basis (Figure 1, Table 1) and by about 16.4% and 43.8%, respectively, when expressed as "ml methane produced per unit mg truly degraded substrate" (Figure 2, Table 1). These two concentrations of *Moringa oleifera* seed proteins increased the partition factor, PF (mg truly degraded substrate/ml produced gas; an index of efficiency of microbial protein synthesis) from 3.18 to 3.24 and 3.47, and decreased the molar proportion of acetate to propionate from 3.11 to 2.7 and 1.8, respectively (Table 2).

Table 1: Effect of crude protein and pure protein on partition factor and methane production

| Treatment* | PF | Met % | MR% | M/TDS | $MR_{TD}$% |
|---|---|---|---|---|---|
| C | 3.18[a] | 17.0[c] | | 0.0533[d] | |
| $CPP_L$ | 3.24[a] | 16.3[c] | 3.0[a] | 0.0503[cd] | 3.9[a] |
| $CPP_H$ | 3.47[a] | 14.4[b] | 14.4[b] | 0.0418[b] | 19.8[b] |
| $PP_L$ | 3.44[a] | 14.8[b] | 12.4[ab] | 0.0439[bc] | 16.4[b] |
| $PP_H$ | 3.96[b] | 11.5[a] | 30.8[c] | 0.0295[a] | 43.8[c] |
| SEM | 0.221 | 1.21 | 2.11 | 0.00121 | 1.34 |

\* Treatment:
with crude protein preparation of 2.5 mg ($CPP_L$) and 5 mg ($CPP_H$)
with pure protein of 1.25 ($PP_L$) and 2.5 mg ($PP_H$)
C = control
PF = partition factor (mg of substrate truly degraded/ml gas)
Met % = methane as percentage of total gas
MR% = Methane reduction on a volume basis
M/TDS = methane produced per unit truly digested substrate (ml/mg)
$MR_{TD}$% = Methane reduction per unit degraded matter
L = low concentration
H = high concentration
SEM = standard error of the mean
[a,b,c,d] Values in the same column with different letters are different at $P<0.05$

Table 2: Effect of crude protein and pure protein on short chain fatty acid production

| Treatment* | Total SCFA | Molar proportion | | | C2:C3 |
|---|---|---|---|---|---|
| | Mmol/syr | C2 | C3 | C4 | |
| C | 1.85[b] | 0.665[d] | 0.213[a] | 0.097[a] | 3.11[d] |
| $CPP_L$ | 1.80[b] | 0.647[c] | 0.235[b] | 0.094[b] | 2.75[c] |
| $CPP_H$ | 1.30[a] | 0.586[b] | 0.240[b] | 0.088[b] | 1.89[b] |
| $PP_L$ | 1.62[b] | 0.637[c] | 0.309[c] | 0.099[c] | 2.64[c] |
| $PP_H$ | 1.28[a] | 0.565[a] | 0.325[d] | 0.107[d] | 1.73[a] |
| SEM | 0.023 | 0.0034 | 0.0012 | 0.0033 | 0.231 |

C2 = acetate
C3 = Propionate
C4 = butyrate
SCFA = short chain fatty acid

An increase in the partition factor from 3.39 to 3.52 and 4.30 was observed in pure protein in concentrations of 1.25 and 2.5 mg per ml, respectively. This pure protein reduced methane production by 12.4% and 34.8% on a volume basis and by 16.4% and 43.8% on basis of truly degraded matter (Table 1). Decreases in molar proportion of acetate to propionate from 3.11 (control) to 2.64 and 1.73 with protein concentrations of 1.25 and 2.5 mg per ml, respectively, were observed with the pure protein (Table 2). The increase in the proportion of propionate and also of the partition factor suggests increased efficiency of rumen fermentation.

The anti-methanogenic activity of the protein was not affected by the heat treatment (100°C for 30 min), indicating good heat stability of the protein.

## 3. Conclusion

The protein has strong anti-methanogenic activity. It has the potential to decrease methane emission from ruminants and at the same time to increase livestock productivity.

**References**

1. Blümmel M. et al., 1997. J. Anim. Phys. Nutr., 77, pp. 24-34
2. Maiworm K., 1994. Dissertation, Tierärzte. Hochschule Hannover, Germany
3. Malik R. and Sirohi S.K., 2000. Intas Polivet, 1, pp. 62-64
4. Menke K.H. and Steingass H., 1988. Anim. Res. Dev., 28, pp. 7-55
5. Moss A., 2000. Ann. Zootech., 49, pp. 231-253
6. Newbold C.M. et al., 1993. J. Appl. Microbiol., 75, pp. 129-134
7. Suarez M. et al., 2005. Antimicrob. Agents and Chemotherapy , 49/5, pp. 3847-3857
8. Nilsson B. et al., 2005. Annu. Rev. Biophys. Biomol. Struct., 34, pp. 91-118
9. Polakovic G., July 13, 2003. The Milwaukee J. Sentinel, LA Times, online
10. Sharma R.K., 2005. J. Res., SKUAST-J., 4/1, pp.1-12
11. van Nevel C.J. and Demeyer D.I., 1996. Environ. Monitoring and Assess- ment.
12. Wallace R.J. et al., 2006. Int. Congress Series, 1293, 42, pp.73-97 pp. 148- 151
13. Wolin, M.J. et al., 1997. In: 'The rumen Microbial Ecosystem', Eds. Hobson P.N. and Stewart C.S., pp.467-491

**Claims**

1. A feed for herbivores, wherein the feed comprises at least one component of a *Moringaceae*.

2. A feed additive for herbivores, wherein the feed additive comprises at least one component of a *Moringaceae*.

3. The feed or feed additive of claim 1 or 2, wherein the component of the *Moringaceae* is selected from the group consisting of chopped components, seeds, natural proteins extracted from the *Moringaceae*, chemically synthesised proteins, proteins expressed by recombination and factors generated by recombination.

4. The feed or feed additive of any of claims 1 to 3, wherein the *Moringaceae* is *Moringa,* preferably *Moringa concanensis, Moringa peregrine, Moringa ovalifolia, Moringa drouhardii, Moringa hildebrandtii* or *Moringa oleifera*.

5. The feed or feed additive of any of claims 1 to 4, wherein the herbivores are ruminants.

6. The feed or feed additive of any of claims 3 to 5, wherein *Moringaceae* proteins are encoded by sequences selected from the group consisting of SEQ ID NO: 1 and 2.

7. The feed or feed additive of any of claims 3 to 5, wherein the factors generated by recombination are selected from the group consisting of ligands, small molecules, co-factors, products of enzymatic reactions, DNAs, RNAs and PNAs.

8. A kit comprising at least one component of a *Moringaceae* for the treatment and/or prevention of gastro-intestinal diseases and/or disorders of herbivores.

9. A method for manufacturing a feed for herbivores comprising the steps of:

   - providing at least one basic feed ingredient;
   - obtaining at least one component of a *Moringaceae*; and
   - mixing at least one component of a *Moringaceae* with at least one basic feed ingredient.

10. A method for manufacturing a feed additive for herbivores comprising the steps of:

    - providing at least one feed additive constituent;
    - obtaining at least one component of a *Moringaceae*; and
    - mixing at least one component of a *Moringaceae* with at least one feed additive constituent.

11. A method for increasing livestock productivity of herbivores comprising the steps of:

    - providing a feed or a feed additive comprising at least one component of a *Moringaceae*; and
    - feeding the feed or the feed additive to herbivores.

12. The method of claim 9, wherein the basic feed ingredient is manufactured from raw material selected from the group consisting of cereals, corn, potatoes, vegetables, legumes, manioc, oily seeds, fish and fibres.

13. The method of claim 10, wherein the feed additive constituent is selected from the group consisting of minerals, trace elements, vitamins, enzymes, immuno modulatory substances and mycotoxin deactivating agents.

14. The method of any of claims 9 to 13, wherein the component of the *Moringaceae* is selected from the group consisting of chopped components, seeds, natural proteins extracted from a *Moringaceae*, chemically synthesised proteins, proteins expressed by recombination and factors generated by recombination.

15. The method of any of claims 9 to 14, wherein the *Moringaceae* is *Moringa,* preferably *Moringa concanensis, Moringa peregrine, Moringa ovalifolia, Moringa drouhardii, Moringa hildebrandtii* or *Moringa oleifera*.

16. The method of any of claims 9 or 15, wherein the feed or the feed additive comprises dosage units.

17. Use of a feed or feed additive comprising at least one component of a *Moringaceae* to increase livestock productivity and/or decrease methane emission from livestock.

**18.** Use of claim 17, wherein the at least one component of a *Moringaceae* is selected from the group consisting of chopped components, seeds, natural proteins extracted from a *Moringaceae*, chemically synthesised proteins, proteins expressed by recombination and factors generated by recombination.

**19.** Use of claim 17 or 18, wherein the feed additive is administered directly to an animal.

A

**Methane Production**

B

**Methane Reduction**

Figure 1

EP 2 025 242 A1

A

Methane Production

B

Methane Reduction

Figure 2

EP 2 025 242 A1

Figure 3

EP 2 025 242 A1

**European Patent**

**Office**

EUROPEAN SEARCH REPORT

Application Number

EP 07 01 4605

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | REYES SANCHEZ ET AL: "Effect of feeding different levels of foliage of Moringa oleifera to creole dairy cows on intake, digestibility, milk production and composition" LIVESTOCK SCIENCE, ELSEVIER, AMSTERDAM, NL, vol. 101, no. 1-3, May 2006 (2006-05), pages 24-31, XP005429797 ISSN: 1871-1413 * the whole document * | 1-6,8-19 | INV. A23K1/00 |
| X | DONGMEZA ET AL: "Effects of dehydrated methanol extracts of moringa (Moringa oleifera Lam.) leaves and three of its fractions on growth performance and feed nutrient assimilation in Nile tilapia (Oreochromis niloticus (L.))" AQUACULTURE, ELSEVIER, vol. 261, no. 1, 6 November 2006 (2006-11-06), pages 407-422, XP005848607 ISSN: 0044-8486 * the whole document * | 1-6, 8-10, 12-16 | |
| X | WO 2007/039911 A (RELIANCE LIFE SCIENCES PVT LTD [IN]; SHAKTI UPADHYAY [IN]; RAMAN YADAV) 12 April 2007 (2007-04-12) * the whole document * | 2-7 | TECHNICAL FIELDS SEARCHED (IPC) A23K C07K |
| A | | 6 | |
| X | WO 03/008441 A (OPTIMA ENVIRONNEMENT S A [CH]; MERMOD NICOLAS [CH]; MARISON IAN WILLIA) 30 January 2003 (2003-01-30) * the whole document * | 2-7 | |
| A | | 6 | |

-/--

The present search report has been drawn up for all claims

| Place of search | Date of completion of the search | Examiner |
|---|---|---|
| Munich | 27 February 2008 | Korb, Margit |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**European Patent Office**

## EUROPEAN SEARCH REPORT

Application Number

EP 07 01 4605

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | SOLIVA C R ET AL: "Feeding value of whole and extracted Moringa oleifera leaves for ruminants and their effects on ruminal fermentation in vitro" ANIMAL FEED SCIENCE AND TECHNOLOGY, ELSEVIER, vol. 118, no. 1-2, 3 January 2005 (2005-01-03), pages 47-62, XP004685866 ISSN: 0377-8401 * the whole document * | 1-6, 8-10, 12-16 | |
| X | HOFFMANN E M ET AL: "EFFECTS OF MORINGA OLEIFERA SEED EXTRACT ON RUMEN FERMENTATION IN VITRO" ARCHIV FUER TIERERNAEHRUNG - ARCHIVES OF ANIMAL NUTRITION, AKADEMIE VERLAG, BERLIN, DE, vol. 57, no. 1, 2003, pages 65-81, XP008052920 ISSN: 0003-942X * the whole document * | 1-6, 8-10, 12-16 | |
| A | WO 2004/050108 A (CELATOUR SOC [FR]; BOUCHER CLAUDE [FR]) 17 June 2004 (2004-06-17) * the whole document * | 1-19 | TECHNICAL FIELDS SEARCHED (IPC) |

The present search report has been drawn up for all claims

| Place of search | Date of completion of the search | Examiner |
|---|---|---|
| Munich | 27 February 2008 | Korb, Margit |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**ANNEX TO THE EUROPEAN SEARCH REPORT
ON EUROPEAN PATENT APPLICATION NO.** EP 07 01 4605

This annex lists the patent family members relating to the patent documents cited in the above-mentioned European search report.
The members are as contained in the European Patent Office EDP file on
The European Patent Office is in no way liable for these particulars which are merely given for the purpose of information.

27-02-2008

| Patent document cited in search report | | Publication date | Patent family member(s) | | Publication date |
|---|---|---|---|---|---|
| WO 2007039911 | A | 12-04-2007 | NONE | | |
| WO 03008441 | A | 30-01-2003 | EP | 1409532 A2 | 21-04-2004 |
| | | | JP | 2005508304 T | 31-03-2005 |
| | | | US | 2004147723 A1 | 29-07-2004 |
| WO 2004050108 | A | 17-06-2004 | AU | 2003294047 A1 | 23-06-2004 |
| | | | EP | 1601369 A1 | 07-12-2005 |
| | | | FR | 2847476 A1 | 28-05-2004 |

EPO FORM P0459

For more details about this annex : see Official Journal of the European Patent Office, No. 12/82

# EP 2 025 242 A1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **BLÜMMEL M. et al.** *J. Anim. Phys. Nutr.,* 1997, vol. 77, 24-34
- **MAIWORM K.** *Dissertation,* 1994
- **MALIK R. ; SIROHI S.K.** *Intas Polivet,* 2000, vol. 1, 62-64
- **MENKE K.H. ; STEINGASS H.** *Anim. Res. Dev.,* 1988, vol. 28, 7-55
- **MOSS A.** *Ann. Zootech.,* 2000, vol. 49, 231-253
- **NEWBOLD C.M. et al.** *J. Appl. Microbiol.,* 1993, vol. 75, 129-134
- **SUAREZ M. et al.** *Antimicrob. Agents and Chemotherapy,* 2005, vol. 49 (5), 3847-3857
- **NILSSON B. et al.** *Annu. Rev. Biophys. Biomol. Struct.,* 2005, vol. 34, 91-118
- **POLAKOVIC G.** *The Milwaukee J. Sentinel, LA Times,* 13 July 2003
- **SHARMA R.K.** *J. Res., SKUAST-J.,* 2005, vol. 4 (1), 1-12
- **VAN NEVEL C.J. ; DEMEYER D.I.** *Environ. Monitoring and Assess- ment,* 1996
- **WALLACE R.J. et al.** *Int. Congress Series,* 2006, vol. 1293 (42), 73-97148-151
- **WOLIN, M.J. et al.** The rumen Microbial Ecosystem. 1997, 467-491

SEQUENCE LISTING

<110> Universität Hohenheim

<120> Feed and feed additive for herbivores, and method for manufacturing the same

<130> T30010

<160> 2

<170> PatentIn version 3.3

<210> 1
<211> 60
<212> PRT
<213> Moringa oleifera

<400> 1

```
Gln Gly Pro Gly Arg Gln Pro Asp Phe Gln Arg Cys Gly Gln Gln Leu
1               5                   10                  15


Arg Asn Ile Ser Pro Pro Gln Arg Cys Pro Ser Leu Arg Gln Ala Val
            20                  25                  30


Gln Leu Thr His Gln Gln Gln Gly Gln Val Gly Pro Gln Gln Val Arg
        35                  40                  45


Gln Met Tyr Arg Val Ala Ser Asn Ile Pro Ser Thr
    50                  55                  60
```

<210> 2
<211> 60
<212> PRT
<213> Moringa oleifera

<400> 2

```
Gln Gly Pro Gly Arg Gln Pro Asp Phe Gln Arg Cys Cys Gln Gln Leu
1               5                   10                  15


Arg Asn Ile Ser Pro Pro Cys Arg Cys Pro Ser Leu Arg Gln Ala Val
            20                  25                  30


Gln Leu Thr His Gln Gln Gln Gly Gln Val Gly Pro Gln Gln Val Arg
        35                  40                  45


Gln Met Tyr Arg Val Ala Ser Asn Ile Pro Ser Thr
    50                  55                  60
```